(19) Europäisches Patentamt
European Patent Office
Office européen des brevets

(11) Veröffentlichungsnummer: **0 002 721**
**B1**

(12) # EUROPÄISCHE PATENTSCHRIFT

(45) Veröffentlichungstag der Patentschrift: **02.09.81**

(51) Int. Cl.³: **C 07 C 109/04,** C 07 C 109/02, C 07 D 209/08

(21) Anmeldenummer: **78101653.0**

(22) Anmeldetag: **13.12.78**

(54) **Verfahren zur Herstellung von Hydrazinen.**

(30) Priorität: **24.12.77 DE 2758027**

(43) Veröffentlichungstag der Anmeldung: **11.07.79 Patentblatt 79/14**

(45) Bekanntmachung des Hinweises auf die Patenterteilung: **02.09.81 Patentblatt 81/35**

(84) Benannte Vertragsstaaten: **CH DE FR GB**

(56) Entgegenhaltungen:
**US-A 3 317 607**
**HOUBEN – WEYL, »Methoden der organischen Chemie«, 4. Auflage, Band X, Teil 2, 1967, GEORG THIEME VERLAG, Stuttgart**

(73) Patentinhaber: **BAYER AG, Zentralbereich Patente, Marken und Lizenzen, D-5090 Leverkusen 1, Bayerwerk (DE)**

(72) Erfinder: **Nofftz, Lothar, Rheinallee 66, D-5090 Leverkusen 1 (DE)**

**EP 0 002 721 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zur Herstellung von Hydrazinen

Gegenstand der Erfindung ist ein Verfahren zur Herstellung von Hydrazinen der allgemeinen Formel

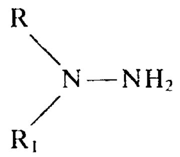

(I)

worin

R einen Aryl- oder Aralkylrest und
$R_1$ einen Alkyl-, Aryl- oder Aralkylrest bedeuten, oder wenn R für einen Arylrest steht,
$R_1$ auch für einen Alkylen- oder Cycloalkylenrest stehen kann, der mit R in der ortho-Stellung zum Stickstoff verbunden ist.

Das Verfahren ist dadurch gekennzeichnet, daß man sekundäre Amine der allgemeinen Formel

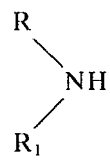

(II)

zu den entsprechenden Nitrosoverbindungen umsetzt, diese ohne Isolierung zunächst bei 0 bis 50° C und bei pH-Werten von 5 bis 6 mit Zinkstaub in Gegenwart von Salzsäure oder Schwefelsäure reduziert und die Reduktion bei pH-Werten unter 4 zu Ende führt.

Unter einem Alkylrest wird insbesondere ein Rest mit 1 bis 12 Kohlenstoffatomen verstanden. Aryl steht vorzugsweise für Phenyl und Naphthyl und Aralkyl für Benzyl oder Phenyläthyl. Die Ringe können durch eine oder zwei $C_1-C_4$-Alkyl-, $C_1-C_4$-Alkoxy- oder Phenylgruppen oder Halogenatome, besonders Chlor oder Brom, substituiert sein.

Bevorzugte Gruppen für $R_1$ = Alkylen oder Cycloalkylen, die mit R in der ortho-Stellung verbunden sind, sind Äthylen-, Propylen(1,3)- und Cyclohexylen(1,2)-Gruppen. Diese Gruppen können durch 1 bis 3 $C_1-C_4$-Alkylrest substituiert sein.

Beispiele für die erfindungsgemäß einzusetzenden Amine sind folgende:

Phenyl-methylamin, Phenyl-äthylamin, Phenyl-propylamin, Phenyl-butylamin, Phenyl-pentylamin, Phenyl-heptylamin, Phenyl-octylamin, Phenyl-nonylamin, Phenyl-decylamin, Phenyl-undecylamin, Phenyl-dodecylamin, 4-Methoxyphenyl-methylamin, 4-Methoxyphenyl-äthylamin, 4-Methoxyphenyl-propylamin, 4-Methoxyphenyl-butylamin, 4-Methoxyphenyl-pentylamin, 4-Methoxyphenyl-hexylamin, 4-Methoxyphenyl-heptylamin, 4-Methoxyphenyl-octylamin, 4-Methoxyphenyl-nonylamin, 4-Methoxyphenyl-decylamin, 4-Methoxyphenyl-undecylamin, 4-Methoxyphenyl-dodecylamin, 4-Äthoxyphenyl-methylamin, 4-Äthoxyphenyl-äthylamin, 4-Äthoxyphenyl-propylamin, 4-Äthoxyphenyl-butylamin, 4-Äthoxyphenyl-pentylamin, 4-Äthoxyphenyl-hexylamin, 4-Äthoxyphenyl-heptylamin, 4-Äthoxyphenyl-octylamin, 4-Äthoxyphenyl-nonylamin, 4-Äthoxyphenyl-decylamin, 4-Äthoxyphenyl-undecylamin, 4-Äthoxyphenyl-dodecylamin, 4-Chlorphenyl-methylamin, 4-Chlorphenyl-äthylamin, 4-Chlorphenyl-propylamin, 4-Chlorphenyl-butylamin, 4-Chlorphenyl-pentylamin, 4-Chlorphenyl-hexylamin, 4-Chlorphenyl-heptylamin, 4-Chlorphenyl-octylamin, 4-Chlorphenyl-nonylamin, 4-Chlorphenyl-decylamin, 4-Chlorphenyl-undecylamin, 4-Chlorphenyl-dodecylamin, 4-Bromphenyl-methylamin, 4-Bromphenyl-äthylamin, 4-Bromphenyl-propylamin, 4-Bromphenyl-butylamin, 4-Bromphenyl-pentylamin, 4-Bromphenyl-hexylamin, 4-Bromphenyl-heptylamin, 4-Bromphenyl-octylamin, 4-Bromphenyl-nonylamin, 4-Bromphenyl-decylamin, 4-Bromphenyl-undecylamin, 4-Bromphenyl-dodecylamin, 4-Methylphenyl-methylamin, 4-Methylphenyl-äthylamin, 4-Methylphenyl-propylamin, 4-Methylphenyl-butylamin, 4-Methylphenyl-pentylamin, 4-Methylphenyl-hexylamin, 4-Methylphenyl-heptylamin, 4-Methylphenyl-octylamin, 4-Methylphenyl-nonylamin, 4-Methylphenyl-decylamin, 4-Methylphenyl-undecylamin, 4-Methylphenyl-dodecylamin, 3,4-Dichlorphenyl-methylamin, 3,4-Dichlorphenyl-äthylamin, 3,4-Dichlorphenyl-propylamin, 3,4-Dichlorphenyl-butylamin, 3,4-Dichlorphenyl-pentylamin, 3,4-Dichlorphenyl-hexylamin, 3,4-Dichlorphenyl-heptylamin, 3,4-Dichlorphenyl-octylamin, 3,4-Dichlorphenyl-nonylamin, 3,4-Dichlorphenyl-decylamin, 3,4-Dichlorphenyl-undecylamin, 3,4-Dichlorphenyl-dodecylamin,

2,4-Dimethylphenyl-methylamin, 2,4-Dimethylphenyl-äthylamin, 2,4-Dimethylphenyl-propylamin, 2,4-Dimethylphenyl-butylamin, 2,4-Dimethylphenyl-pentylamin, 2,4-Dimethylphenyl-hexylamin, 2,4-Dimethylphenyl-heptylamin, 2,4-Dimethylphenyl-octylamin, 2,4-Dimethylphenyl-nonylamin, 2,4-Dimethylphenyl-decylamin, 2,4-Dimethylphenyl-undecylamin, 2,4-Dimethylphenyl-dodecylamin, Diphenylamin, Phenyl-benzylamin, 4-Methoxyphenyl-benzylamin, 4-Chlorphenyl-benzylamin, 1,2,3,4-Tetrahydrochinolin, 1,2,3,4-Tetrahydro-6-methoxychinolin, 2,3,3-Trimethyl-2,3-dihydroindol, 2,3,3-Trimethyl-5-chlor-2,3-dihydroindol, 2,3,3-Trimethyl-5-brom-dihydroindol, 2,3,3-Trimethyl-5-methoxy-2,3-dihydroindol, 2,3,3-Trimethyl-5-äthoxy-2,3-dihydroindol, 2-Methyl-2,3-dihydroindol, 2-Methyl-5-methoxy-2,3-dihydroindol, 2-Methyl-5-äthoxy-2,3-dihydroindol, 2-Methyl-5-chlor-2,3-dihydroindol, 2-Methyl-5-brom-2,3-dihydroindol, 2,3,3,5-Tetramethyl-2,3-dihydroindol, 2,3,3,5,7-Pentamethyl-2,3-dihydroindol, 2,3,3-Trimethyl-5-äthyl-2,3-dihydroindol, 2,5-Dimethyl-2,3-dihydroindol, 2-Methyl-5-äthyl-2,3-dihydroindol, 2,2,4-Trimethyl-1,2,3,4-tetrahydrochinolin und Hexahydrocarbazol.

Die Reduktion der sich von den Aminen (II) ableitenden Nitrosamine mit Zink wurde bisher in essigsaurem Medium durchgeführt (z. B. Houben – Weyl, »Methoden der organischen Chemie«, Bd. X/2 (1967), S. 224). Die Reduktion in Gegenwart von Salzsäure führte nach den dort gemachten Angaben stets zu einer Rückspaltung der Nitrosoverbindungen zu den Aminen.

Es wurde nun überraschend gefunden, daß die Hydrazine (I) in ausgezeichneter Ausbeute und hoher Reinheit durch Reduktion mit Zink in Gegenwart von Salz- oder Schwefelsäure nach dem neuen Verfahren erhalten werden können.

Die Nitrosierung der Amine wird nach bekannten Verfahren in wäßrigem, alkoholischem oder wäßrig-alkoholischem Medium durchgeführt.

Die Reaktion wird bevorzugt mit einem Überschuß an Natriumnitrit, z. B. einem Überschuß von 2 bis 7%, durchgeführt.

Die Konzentration der Amine kann in weiten Grenzen schwanken. Sie ist nach oben begrenzt durch die Rührbarkeit der Suspension des Aminsalzes bzw. des Nitrosamins. Eine untere Grenze ist dadurch gegeben, daß die vollständige Ausfällung des erhaltenen Hydrazinsalzes aus einer sehr verdünnten Lösung Schwierigkeiten bereitet.

Als technisch vorteilhaft hat sich erwiesen, die Konzentration so zu wählen, daß die Konzentration des Nitrosamins bei 7 bis 25% liegt.

Die bei der Nitrosierung erhaltene salz- oder schwefelsaure Reaktionsmischung wird zur Reduktion mit Zinkstaub versetzt. Dabei wird insbesondere eine Zinkmenge von 2 bis 5 Mol pro Mol Nitrosamin verwendet.

Die Zugabe des Zinks erfolgt so, daß die Temperatur 50° C nicht übersteigt. Die Temperatursteuerung kann durch Kühlung oder durch die Geschwindigkeit der Zinkzugabe erfolgen.

Vorzugsweise verfährt man so , daß man die Reaktionsmischung vor der Zugabe des Zinks auf 0 bis 5° C kühlt und nach Beendigung der Zugabe die Temperatur auf 40 bis 50° C ansteigen läßt.

Bei der Zugabe des Zinks erhöht sich der pH-Wert von etwa 0 bis 1 auf etwa 5 bis 6. Anschließend wird dem Gemisch Salz- oder Schwefelsäure zugefügt unter Erhaltung eines pH-Wertes von 5 bis 6 und Einhaltung einer Temperatur bis zu 50° C. Nachdem der größte Teil des Zinks verbraucht ist, wird die Zugabe der Säure fortgesetzt, bis ein pH-Wert von unter 4, vorzugsweise von 1 bis 2, erreicht wird.

## Beispiel 1

In einem Rührkolben, aus dem mit Stickstoff die Luft verdrängt war, wurden in

| | |
|---|---|
| 133 ml | Wasser |
| 107 g | N-Methylanilin (1,0 Mol) mit |
| 169 g | 30%iger Salzsäure (1,54 Mol) klar gelöst. Bei 0 bis 5° C lief in 30 Minuten eine Lösung von |
| 71,4 g | Natriumnitrit (1,03 Mol) in |
| 150 ml | Wasser in obige Lösung ein. Es wurde 30 Minuten nachgerührt, wobei das Nitrosamin feinkristallin ausfiel. Bei 0° C wurden in das Reaktionsgemisch |
| 162 g | Zinkstaub (2,5 Mol) eingetragen und die Außenkühlung entfernt. Die Temperatur stieg auf 36° C und der pH-Wert auf 6,0. Nun liefen in ca. 4 Stunden bei 40 bis 45° C unter leichter Kühlung |
| 837 g | 30%ige Salzsäure (6,9 Mol) ein. Es wurde darauf geachtet, daß zunächst der pH-Wert nicht unter 5 absank. Nachdem ca. 2/3 der Salzsäuremenge zugelaufen war, sank der pH-Wert langsam ab und erreichte zum Schluß pH 1. Es wurde über Nacht bei 35 bis 40° C nachgerührt, wobei fast alles in Lösung ging. Der Lösung wurden |

6 g A-Kohle zugesetzt. Dann wurde durch Zugabe von
70 g Natronlauge 50% (0,9 Mol) auf pH 4,3 gestellt und bei 30°C abgenutscht. Das Produkt wurde auf der Nutsche gut trockengesaugt und anschließend im Vakuum bei 40°C getrocknet. Es wurden
121,6 g N-Methyl-N-phenylhydrazin-Hydrochlorid (0,77 Mol) gewonnen, das noch ca. 3 bis 5% N-Methylanilin enthielt.

Das Hydrochlorid läßt sich durch Behandeln in einer wäßrigen Anschlämmung mit Natronlauge in die freie Base überführen, die sich als Öl abscheidet. Eine Reinigung kann beispielsweise über das Sulfat mittels Alkohol erfolgen, wie sie von E. Fischer (Ann. 190, 154) beschrieben wird.

Aus der wäßrigen Phase lassen sich durch Einstellen des pH-Wertes auf 9 38,6 g eines Öls erhalten, das laut Gaschromatogramm 30% N-Methyl-N-phenylhydrazin und 70% N-Methylanilin enthielt.

Bei den nachfolgenden Beispielen wurde ebenfalls unter Ausschluß von Luftsauerstoff und mit einem geringen Nitritüberschuß bei der Nitrosierung gearbeitet. Die pH-Werte entsprechen ebenfalls Beispiel 1.

## Beispiel 2

169 g Diphenylamin (1,0 Mol) wurden in
600 ml Alkohol gelöst und bei 15 bis 20°C in 30 Minuten mit
238 g 37%iger Salzsäure (1,21 Mol) versetzt. Es entstand ein gut rührbarer Brei, in den bei 0 bis 5°C in einer Stunde eine Lösung von
71,4 g Natriumnitrit (1,03 Mol) in
150 ml Wasser eingeleitet wurde. Es wurde eine Stunde nachgerührt (pH1).
Bei 0°C wurden
248 g Zinkstaub (3,8 Mol) zugesetzt und 15 Minuten nachgerührt. Der pH-Wert stieg auf 5,0 und die Temperatur auf 30°C. In 4 Stunden wurden bei 40 bis 45°C
642 g Salzsäure 37% (6,52 Mol) eingeleitet und über Nacht gerührt. Es entstand eine fast klare Lösung, die mittels A-Kohle geklärt wurde. Die klare Lösung wurde mit Wasser und Eis auf 5000 ml verdünnt und mit
714 g Salzsäure 37% (7,2 Mol) versetzt.
Nachdem die Ausfällung eine Stunde bei 0°C verrührt worden war, wurde das Produkt abgenutscht und bei 40°C im Vakuum getrocknet. Es wurden 193,8 g trockenes N,N-Diphenylhydrazin-Hydrochlorid (0,88 Mol) gefunden, die nach Dünnschichtchromatogramm noch 3 bis 5% Diphenylamin enthielten.
Das Produkt wurde bei 80°C und pH1 in salzsaurem Wasser gelöst, Diphenylamin abfiltriert und das reine Produkt durch Salzsäurezusatz bei 0°C als Hydrochlorid gefällt.

Elementaranalyse:

| | | | | |
|---|---|---|---|---|
| berechnet: | 65,3% C | 5,90% H | 12,70% N | 16,10% Cl |
| gefunden: | 64,95% C | 6,05% H | 12,55% N | 16,10% Cl |

## Beispiel 3

183,3 g N-Phenylbenzylamin (1 Mol) wurden in
2000 ml Wasser bei 20 bis 25°C innerhalb 30 Minuten mit
345 g Salzsäure 30% (2,83 Mol) gelöst. Bei 0 bis 5°C wurde in 30 Minuten eine Lösung von
71,4 g Natriumnitrit (1,03 Mol) in
150 ml Wasser einlaufen gelassen und eine Stunde nachgerührt. Es lag ein gut rührbarer Kristallbrei mit pH 0,5 vor. Es wurden bei 0°C
300 g Zinkstaub (4,6 Mol) eingetragen und 30 Minuten nachgerührt. Die Temperatur stieg auf 35°C und der pH-Wert auf 6,0. Es wurden in 4 Stunden bei 40 bis 45°C
1150 g Salzsäure 30% (9,45 Mol) einlaufen gelassen. Es entstand zunächst eine klare Lösung, aus der ab pH 2 das entstandene Produkt in weißen Kristallen ausfiel. Es wurde 3 Stunden nachgerührt und die Ausfällung auf 20°C abgekühlt. Sie wurde abgenutscht und der Rückstand bei 80°C in
2000 ml Wasser mit Salzsäure bei pH 0,5 gelöst, mit
10 g A-Kohle 15 Minuten verrührt und filtriert. Die klare Lösung wurde mit
300 g Natriumchlorid versetzt und auf 10°C abgekühlt.
Das hierbei ausgefallene Produkt wurde abgenutscht und im Vakuum bei 50°C getrocknet. Gewonnen wurden 205,6 g N-Phenyl-N-benzylhydrazin als Hydrochlorid (0,875 Mol).

Elementaranalyse:

| | | | | |
|---|---|---|---|---|
| berechnet: | 66,53% C | 6,40% H | 11,94% N | 15,14% Cl |
| gefunden: | 66,35% C | 6,85% H | 12,00% N | 15,10% Cl |

Beispiel 4

| | |
|---|---|
| 133 g | 2-Methylindolin (1 Mol) wurden in |
| 133 ml | Wasser bei 15 bis 20° C in 30 Minuten mit |
| 170 g | Salzsäure 30% (1,4 Mol) klar gelöst. Bei 5 bis 10° C lief eine Lösung von |
| 72,45 g | Natriumnitrit (1,05 Mol) in |
| 150 ml | Wasser in 30 Minuten in die Lösung ein. Der hierbei entstandene, gut rührbare Kristallbrei wurde bei pH 0 noch eine halbe Stunde verrührt und auf 0° C abgekühlt. Danach wurden |
| 163 g | Zinkstaub (2,5 Mol) zugesetzt. Innerhalb von 20 Minuten stiegen die Temperatur auf 35° C und der pH-Wert auf 6,0 an. Danach liefen in 3 Stunden bei 40 bis 45° C |
| 841,5 g | Salzsäure 30% (6,9 Mol) in das Reaktionsgemisch ein. Nach beendetem Einlauf wurden der entstandenen Lösung |
| 6 g | Kieselgur zugesetzt und über Nacht bei 35 bis 40° C verrührt. Am nächsten Morgen wurde die Lösung vom Rückstand, der kein metallisches Zink mehr enthielt, abfiltriert. Die klare Lösung wurde auf 0 bis 1° C abgekühlt und 3 Stunden bei dieser Temperatur verrührt. Das hierbei ausgefallene weiße kristalline Produkt wurde abgenutscht und auf der Nutsche mit einem Gemisch von |
| 150 ml | Wasser, |
| 30 g | Kochsalz und |
| 35 ml | Salzsäure 30%, das auf 0° C abgekühlt war, nachgewaschen und danach gut trockengesaugt. Danach wurde das Produkt im Vakuum bei 60° C getrocknet. Es wurden 150,7 g 1-Amino-2-methylindolin als Hydrochlorid erhalten. Das Produkt war 94,1%ig, entsprechend 141,7 g (0,768 Mol) 100%igem Produkt. Mit Hilfe eines Dünnschichtchromatogramms wurden ca. 0,05% einer unbekannten Verbindung und ca. 0,8% 2-Methylindolin als organische Verunreinigung gefunden. |

Beispiel 5

| | |
|---|---|
| 133 g | 2-Methylindolin (1,0 Mol) wurden in |
| 133 ml | Wasser bei 15 bis 20° C mit |
| 216 g | Schwefelsäure 50% (1,1 Mol) gelöst. Bei 0 bis 5° C wurden in 30 Minuten |
| 72,45 g | Natriumnitrit (1,05 Mol) gelöst in |
| 150 ml | Wasser, eingeleitet. Bei 0° C wurden |
| 143 g | Zinkstaub (2,2 Mol) eingetragen. Innerhalb von 20 Minuten stiegen die Temperatur auf 35° C und der pH-Wert auf 6,0 an. In 4 Stunden liefen |
| 650 g | Schwefelsäure 50% (3,3 Mol) bei 40 bis 45° C ein. Es wurde über Nacht bei 35 bis 40° C nachgerührt. Anschließend wurde zur völligen Lösung der ausgefallenen Kristalle auf 80° C erhitzt. Es wurden |
| 6 g | A-Kohle zugesetzt, 15 Minuten verrührt und bei 80° C klar filtriert. Die klare Lösung wurde auf 0° C abgekühlt, eine Stunde verrührt, abgenutscht und mit einem Gemisch von |
| 150 ml | Wasser, |
| 20 g | Natriumsulfat und |
| 25 ml | Schwefelsäure 50% bei 0° C gewaschen. Die weißen Kristalle wurden bei 60° C im Vakuum getrocknet. Es wurden 304,3 g 1-Amino-2-methylindolin als schwefelsaures Salz gefunden. |

**Patentansprüche**

1. Verfahren zur Herstellung von Hydrazinen der allgemeinen Formel

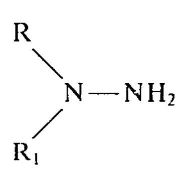

worin

R einen Aryl- oder Aralkylrest und
$R_1$ einen Alkyl-, Aryl- oder Aralkylrest bedeuten, oder wenn R für einen Arylrest steht,
$R_1$ auch für einen Alkylen- oder Cycloalkylenrest stehen kann, der mit R in der ortho-Stellung zum Stickstoff verbunden ist,

dadurch gekennzeichnet, daß man sekundäre Amine der allgemeinen Formel

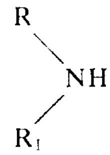

zu Nitrosaminen umsetzt und diese ohne Isolierung bei 0 bis 50° C und bei pH-Werten von 5 bis 6 mit Zinkstaub in Gegenwart von Salzsäure oder Schwefelsäure reduziert und die Reduktion bei pH-Werten von unter 4 zu Ende führt.

2. Verfahren nach Anspruch 1, dadurch gekennzeichnet, daß man den Zinkstaub zu einer Reaktionsmischung gibt, die neben dem Nitrosamin einen Überschuß an Natriumnitrit enthält.

3. Verfahren nach Anspruch 1, dadurch gekennzeichnet, daß man den Zinkstaub zu einer Reaktionsmischung, die auf 0 bis 5° C gekühlt wurde, gibt und die Temperatur während der Zugabe des Zinkstaubs auf 40 bis 50° C ansteigen läßt.

**Claims**

1. Process für the preparation of hydrazines of the general formula

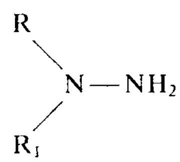

wherein

R denotes an aryl or aralkyl radical and
$R_1$ denotes an alkyl, aryl or aralkyl radical, or, if R represents an aryl radical
$R_1$ can also represent an alkylene or cycloalkylene radical which is bonded to R in the ortho-position relative to the nitrogen,

characterised in that secondary amines of the general formula

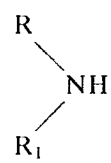

are reacted to give nitrosamines and these are reduced, without being isolated, with zinc dust in the presence of hydrochloric acid or sulphuric acid at 0 to 50° C and at pH values of 5 to 6, and the reduction is brought to completion at pH values of less than 4.

2. Process according to Claim 1, characterised in that the zinc dust is added to a reaction mixture which contains, in addition to the nitrosamine, an excess of sodium nitrite.

3. Process according to Claim 1, characterised in that the zinc dust is added to a reaction mixture which has been cooled to 0 to 5° C, and the temperature is allowed to rise to 40 to 50° C during the addition of the zinc dust.

**Revendications**

1. Procédé de production d'hydrazines de formule générale:

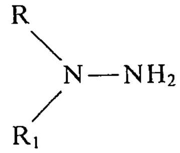

dans laquelle

R est un reste aryle ou aralkyle et
$R_1$ est un reste alkyle, aryle ou aralkyle, ou bien lorsque R est un reste aryle,
$R_1$ peut aussi représenter un reste alkylène ou cycloalkylène qui est lié à R en position ortho par rapport à l'azote,

caractérisé en ce qu'on transforme des amines secondaires de formule générale:

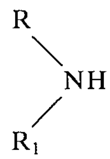

en nitrosamines et on réduit ces dernières sans les isoler à une température de 0 à 50° C et à des valeurs de pH de 5 à 6 avec du zinc en poudre en présence d'acide chlorhydrique ou d'acide sulfurique et on achève la réduction à des valeurs de pH inférieures à 4.

2. Procédé suivant la revendication 1, caractérisé en ce qu'on ajoute du zinc en poudre à un mélange réactionnel qui contient, à côté de la nitrosamine, un excès de nitrite de sodium.

3. Procédé siuvant la revendication 1, caractérisé en ce qu'on ajoute le zinc en poudre à un mélange réactionnel qui a été refroidi à une température de 0 à 5° C, et on fait croitre la température pendant l'addition du zinc en poudre à une température de 40 à 50° C.